# DEMANDE DE BREVET EUROPEEN

(11) **EP 2 813 217 A1**
(43) Date de publication de la demande: **17.12.2014**
(21) Numéro de dépôt: 13305789.3
(22) Date de dépôt: 11.06.2013
(51) Int. Cl.: A61K 9/28, A61K 33/06, A61K 33/14, A61K 45/06

(54) **Composition pour administration orale de magnésium, en association avec une composition destinée à traiter le diabète de type 2 ou ses complications**

(71) Demandeur: Joanny Menvielle-Bourg, Fabienne, 06400 Cannes (FR)
(72) Inventeur: Joanny Menvielle-Bourg, Fabienne, 06400 Cannes (FR)
(74) Mandataire: Tezier Herman, Béatrice

(57) **Abrégé**

La présente invention a trait à une composition particulière pour une administration orale comprenant du magnésium, pour une utilisation dans le traitement du diabète de type 2 ou ses complications, en association avec une composition destinée à traiter le diabète de type 2 ou ses complications.

## Description

### Domaine de l'invention

La présente invention a trait à une composition pour une administration orale comprenant du magnésium, pour une utilisation dans le traitement du diabète de type 2 ou ses complications, en association avec une composition destinée à traiter le diabète de type 2 ou ses complications. La composition comprenant du magnésium est plus spécifiquement sous la forme de comprimé à libération prolongée.

### Art antérieur

On sait, notamment des demandes de brevets WO 01/22943, US 6887492, US 5849338 et GB 1356097 illustrant l'état antérieur de la technique, que l'on a déjà proposé dans le passé des solutions techniques destinées à fournir du magnésium à libération décalée dans le temps.

Par ailleurs, en ce qui concerne les compositions orales de magnésium à libération prolongée, on connaît le brevet européen EP 0542979 et les demandes internationales WO 2004/105778 et WO 2009/150323.

Le magnésium, sous forme d'hydrate, d'oxyde hydraté, de carbonate, de chlorure et d'autres sels encore, est très utilisé sous forme de compléments alimentaires et/ de médicaments. L'ion magnésium Mg²⁺ joue un rôle très important dans l'équilibre ionique du corps humain, en particulier il joue un rôle dans de nombreuses réactions enzymatiques impliquées dans des processus métaboliques chez l'homme. Les taux de magnésium dans le plasma de personnes en bonne santé sont extrêmement constants, avec un intervalle de référence pour des taux de sérum total de 0,75-0,96 mmol/L. Un certain nombre d'hormones incluant l'hormone parathyroïde (PTH) et calcitonine, vitamine D, glucagon, hormone antidiurétique, aldostérone, stéroïdes sexuels et l'insuline ont été décrites comme influençant la balance du magnésium (N E Saris et al. Magnesium. An update on Physiological, Clinical and Analytical aspects, Clinica chimica acta ; International Journal of Clinical Chemistry, 294 (2000), 1-26). L'insuline stimule la réabsorption rénale du magnésium dans la branche ascendante de l'anse de Henle. Ainsi, la déficience en insuline affecte directement le transport du magnésium en plus de développer une kétoacidose qui inhibe la conservation du magnésium (G A Quamme, Renal Magnésium Handling : New insights in Understanding Old problems, Kidney international, 52 (1997), 1180-1195).

De nombreux troubles peuvent être associés à un manque de magnésium : dépression et angoisse, diabète, spasmes musculaires, crampes, troubles cardiovasculaires, pression artérielle élevée, insomnie et ostéoporose. Le magnésium participe activement à la transmission des influx nerveux entre les neurones. L'apport recommandé quotidien de magnésium est estimé à 375 mg par jour (le double pour les sportifs ou les femmes enceintes) ou encore environ 6 mg par kg de poids corporel.

La prise de supplément en magnésium est courante sous la forme de médicaments ou de compléments alimentaires. Ces derniers utilisent différents sels (par exemple : chlorure, carbonate, pidolate, aspartate, citrate, lactate, malate, thréonate) ou oxydes et sont présentés sous différentes formes galéniques (par exemple solution, gélule, comprimé) avec dans leur composition un ou plusieurs de ces sels. Ces différentes formes galéniques sur le marché peuvent proposer différents types de libération du magnésium dans l'organisme : à libération immédiate, décalée dans le temps ou bien à libération prolongée.

Le corps ne produit pas de magnésium. Il en consomme plus ou moins vite suivant l'activité physique et le stress. Il doit donc le puiser dans l'alimentation au quotidien et dans des compléments de cette alimentation si nécessaire. La consommation excessive de magnésium est éliminée naturellement par l'organisme dans les urines. Le magnésium ne s'accumule donc pas. L'ingestion de grandes quantités de magnésium provoque un effet laxatif, voire purgatif. L'empoisonnement par excès de magnésium peut exister chez l'enfant et dans le cas de personne souffrant d'insuffisance rénale.

Le diabète mellitus ou plus généralement le diabète est une maladie causée par une déficience ou une diminution de l'efficacité de l'insuline endogène. Cette maladie métabolique est caractérisée par un taux de sucre ou de glucose dans le sang élevé soit parce que le pancréas ne produit pas assez d'insuline (diabète de type 1) soit parce que les cellules ne répondent pas correctement à l'insuline produit (diabète de type 2).

De nombreux résultats suggèrent qu'une prise de magnésium plus élevée peut réduire l'incidence du diabète. Une méta-analyse réalisée par Dong et al. a permis de fournir des preuves comme quoi la prise de magnésium est inversement proportionnelle au risque de développer un diabète de type 2 et ceci de manière dose dépendante (Jia-Yi Dong et al. « Magnésium intake and risk oftype 2 diabetes: meta-analysis of prospective cohort studies, diabetes care, 34 (2011), 2116-2122). Ceci suggère que le magnésium est un des éléments nutritifs impliqués dans la prévention du diabète. Le lien entre le magnésium et le diabète mellitus a donc déjà été décrit. Des études ont montré que les taux moyens de magnésium libre plasmatique et intracellulaire sont plus bas chez les patients diabétiques que la population en général. L'hypomagnésémie survient dans 13,5 % à 47,7% des patients avec du diabète de type 2 comparé à 2,5% à 15% des personnes sans diabète. Des patients avec le diabète mellitus présentent souvent une déficience en magnésium, ce qui pourrait être associé à une résistance à l'insuline (caractéristique des patients atteints de diabète de type 2). Il a également été constaté que le magnésium améliore la réponse à l'insuline au sucre alimentaire et améliore l'action de l'insuline en régulant le taux de sucre dans le sang.

En ce qui concerne les complications du diabète comme les maladies cardiovasculaires, il a été montré que des compléments en magnésium avec MgCl2 permet de réduire la pression sanguine chez les adultes hypertendus diabétiques avec de l'hypomagnésémie.

En ce qui concerne la rétinopathie causée par le diabète, certaines études ont montré que des patients avec une rétinopathie présentent une concentration moyenne plasmatique en magnésium plus basse que chez les patients sans rétinopathie (P McNair and others, 'Hypomagnesemia, a Risk Factor in Diabetic Retinopathy', Diabetes, 27 (1978), 1075-1077).

La néphropathie est une des complications les plus fréquentes du diabète. Il a été décrit que des taux peu élevés en magnésium constatés dans le sérum peuvent être associés à un déclin plus rapide de la fonction rénale chez les patients présentant un diabète de type 2 (P C Pham and others, 'The Link Between Lower Serum Magnésium and Kidney Function in Patients with Diabetes Mellitus Type 2 Deserves a Closer Look', Clinical nephrology, 71 (2009), 375-379 ; P C T Pham and others, 'Lower Serum Magnésium Levels Are Associated with More Rapid Decline of Renal Function in Patients with Diabetes Mellitus Type 2', Clinical nephrology, 63 (2005), 429-436).

Des chercheurs ont montré l'efficacité et l'innocuité de MgCl₂ pour le traitement de la dépression chez les patients âgés avec du diabète de type 2 et une hypomagnésémie (Lazaro Barragán-Rodríguez, Martha Rodríguez-Morán and Fernando Guerrero-Romero, 'Efficacy and Safety of Oral Magnesium Supplementation in the Treatment of Depression in the Elderly with Type 2 Diabetes: a Randomized, Equivalent Trial', Magnesium research: official organ of the International Society for the Development of Research on Magnesium, 21 (2008), 218-223).

Il a aussi été observé qu'une carence en magnésium est présente et reliée à la présence d'ulcères aux pieds chez les patients ayant un diabète de type 2 (M Rodríguez-Morán and F Guerrero-Romero, 'Low Serum Magnesium Levels and Foot Ulcers in Subjects with Type 2 Diabetes', Archives of medical research, 32 (2001), 300-303).

Il est donc raisonnable de penser que la satiété en magnésium peut permettre de retarder l'apparition du diabète, d'en diminuer ses effets et potentiellement de prévenir ses complications graves, telles que les maladies cardiovasculaires, la rétinopathie, néphropathie, dépression et ulcères du pied.

Ainsi, un complément en magnésium chez les patients avec du diabète de type 2 pourrait éventuellement être bénéfique. Cependant, un traitement à long terme avec une dose élevée en magnésium peut causer des effets secondaires qui sont cliniquement pertinents pour l'observance du patient, en particulier chez les patients souffrant d'insuffisance rénale.

Malgré les progrès remarquables réalisés dans le domaine du diabète et ses complications, il reste nécessaire de trouver de nouveaux traitements ou associations médicamenteuses à la fois efficace pour le traitement du diabète et présentant des effets secondaires et/ou toxiques minimisés. Le diabète est une maladie chronique pouvant se maîtriser. La maladie représente une perte de liberté avec un régime strict à suivre, une perte de sa sécurité (exemple crise d'hypoglycémie) et des complications imprévisibles. Pour limiter le risque de complications, il faut scrupuleusement contrôler son diabète, suivre son traitement et les conseils de son médecin. Ainsi, le magnésium est un des éléments qui pourraient permettre d'améliorer la qualité de vie du patient diabétique. Il existe cependant beaucoup de formules ou compositions à base de magnésium (médicaments ou compléments alimentaires) sur le marché actuel mais ils peuvent ne pas être tous adaptés aux besoins du patient diabétique.

A ce titre, il a été identifié par la Demanderesse que l'association d'une composition particulière à base de magnésium et d'une composition pharmaceutique comprenant au moins un actif pharmaceutique pour traiter le diabète de type 2 ou ses complications permet d'obtenir un traitement thérapeutique pour le diabète de type 2 et ses complications particulièrement efficace, sans présenter d'effets secondaires liés notamment à la prise de magnésium. La composition qui sera décrite ci-dessous présente de nombreux avantages, puisqu'elle permet notamment de ne pas interagir avec le traitement du diabète, d'être efficace dans l'amélioration des paramètres physiologiques liés au diabète, de pouvoir être utilisée à longue durée pour corriger une déficience magnésique (permettant en particulier un niveau plasmatique du Mg constant sans pic sanguin), d'avoir une bonne biodisponibilité, d'être bien tolérée au niveau intestinal et sans effets secondaires, de présenter une formulation chimique et galénique diminuant l'élimination rénale du Mg, et de présenter une formulation permettant de diminuer des risques d'hypermagnésémie en cas de néphropathie ou d'insuffisance rénale.

Ainsi, la demanderesse propose une composition comprenant du magnésium à faible dose et à libération prolongée, pour une utilisation pour le traitement du diabète de type 2 ou l'une de ses complications, en association avec une composition pharmaceutique comprenant au moins un actif destiné au traitement du diabète de type 2. Ainsi, la composition particulière comprenant le magnésium permet de maintenir un taux en magnésium relativement constant dans le corps (par exemple, tout le long dudit traitement, jours et nuits), sans provoquer d'effets secondaires dus notamment à des doses trop élevées en magnésium dans le sang.

### Objet de l'invention

Selon un premier aspect de l'invention, on propose une composition comprenant du magnésium à faible dose et à libération prolongée, pour une utilisation pour le traitement du diabète de type 2 ou l'une de ses complications, en association avec une composition pharmaceutique comprenant au moins un actif destiné au traitement du diabète de type 2. Plus spécifiquement, lesdites compositions sont administrées de manière simultanée, séparée ou étalée dans le temps.

Plus spécifiquement, la composition comprenant du magnésium est une composition pour une administration orale, sous forme de comprimé, comprenant une matrice de magnésium avec libération progressive qui forme un noyau constitué d'un agent de retard hydrophile (B1), d'un agent de retard hydrophobe (B2), d'une charge inerte (C1) intervenant en tant que diluant et d'une charge inerte (C2) intervenant en tant que moyen lubrifiant, ladite matrice étant caractérisée en ce qu'elle comprend :
(A) 90 à 110 parties en poids de magnésium, la source de magnésium étant présente sous la forme choisie parmi MgO, MgCl₂ et les hydrates de formule MgCl₂.n(H₂O) où n est un nombre réel supérieur à 0 et inférieur ou égal à 6,
(B1) 180 à 190 parties en poids d'hydroxypropylméthylcellulose,
(B2) 19,8 à 22,2 parties en poids de béhénate de glycéryle,
(C1) 10 à 12 parties en poids de lactose, et
(C2) 10 à 12 parties en poids de silice colloïdale ; ladite composition comprend également un enrobage, qui n'est pas un enrobage gastrorésistant, mais un enrobage de protection ralentissant la dissolution du Mg au niveau gastrique.

L'enrobage de la matrice (i. e. l'enrobage du comprimé nu) n'est pas gastrorésistant. Il intervient pour la protéger, notamment au cours du conditionnement et du stockage, d'une part, et il sert à ralentir la libération du magnésium au niveau gastrique, d'autre part. Selon un mode particulier, il représente 15 à 75 parties en poids pour une quantité de 90 à 110 parties en poids de Mg (i. e. approximativement 1,3 à 7,5 % en poids par rapport au poids de la matrice, c'est-à-dire du comprimé nu).

On a déterminé *in vitro* le taux de dissolution (δ) du magnésium (exprimé en % par rapport au magnésium total apporté par la source de magnésium). La matrice enrobée selon l'invention fournit, après 2h en milieu HCl 0,1N, un taux de dissolution δ qui est inférieur ou égal à 60 %, de préférence entre 20% et 60%.

Quand on souhaite apprécier la cinétique globale de dissolution *in vitro* d'un comprimé, on met en oeuvre un système de dissolution classique (désigné ici «système de dissolution A») d'abord dans un milieu acide [avantageusement 900 ml selon la pharmacopée américaine] HCl 0,1N de T = 0 à T = 2h (i. e. traitement correspondant approximativement à la durée de transit dans l'estomac), puis dans un tampon [avantageusement 900 ml] à pH 6,8 de T = 2h à T = 8h, afin de déterminer les teneurs cumulées en substance active dissoute, ici le magnésium, aux instants T = 2h, T = 4h, T = 6h et T = 8h [i. e. traitement correspondant approximativement à la durée de transit dans l'intestin grêle (de T = 2h à T = 4h) puis traitement correspondant approximativement au transit dans le gros intestin (de T = 4h à T = 8h)]. Cette cinétique de dissolution est déterminée à une température pouvant se situer de la température ambiante (15-25 °C) jusqu'à 40 °C. Comme dans la présente invention le comprimé enrobé et ses constituants sont tous stables au stockage pendant plusieurs mois à 40 °C, la cinétique de dissolution a été mesurée ici à 40 °C par commodité pour se placer dans des conditions de température sensiblement proches de celle de l'intérieur du corps humain.

Le comprimé enrobé utilisé dans la composition selon l'invention présente un profil de dissolution tel que :
- à T = 2h, on a : δ ≤, 60 %, plus précisément : 20 % ≤ δ ≤ 60 %, et de préférence : 25 % ≤ δ ≤ 58 % ;
- à T = 4h, on a : δ ≤ 85 %, plus précisément : 40 % ≤ δ ≤ 85 %, et de préférence : 45 % ≤ δ ≤ 82 % ;
- à T = 6h, on a : δ ≤ 98 %, plus précisément : 60 % ≤ δ ≤ 98 %, et de préférence : 80% ≤ δ ≤ 95% ; et
- à T = 8h, on a : δ ≤ 100 %, plus précisément : 90 % ≤ δ ≤ 100 %, et de préférence : 95 % ≤ δ ≤ 99,9 %.

Ainsi, la composition comprenant le magnésium utilisée dans la présente invention, présente avantageusement une dissolution qui (i) commence dans la phase 'gastrique' (de T = 0 à T = 2h) avec une cinétique de dissolution ralentie [le taux de dissolution (δ) du magnésium par rapport au magnésium administré par le biais de la source de magnésium] étant inférieur ou égal à 60 %], ce qui permet au magnésium d'arriver à dose filée au niveau de l'intestin grêle où il commence à être absorbé avec une cinétique faible (de T = 2h à T = 4h), d'une part, puis d'arriver dans la phase 'gros intestin' (de T =4h à T = 8h), d'autre part. La composition utilisée selon l'invention présente donc l'avantage d'être à libération prolongée. Le terme « libération prolongée » ou « libération progressive » du magnésium correspond donc à une libération en continu du magnésium sous forme biologique ; l'intégralité étant absorbée par l'organisme sur une durée de 8 heures à partir de l'ingestion de comprimé.

Ainsi, la composition comprenant le magnésium présente l'avantage d'une absorption préférentielle de Mg²⁺ au niveau de l'iléon, lieu où l'absorption du Mg est maximale, et une dissolution plus lente et progressive, qui est programmée, de la sortie de l'estomac jusqu'au gros intestin. La libération du magnésium sous la forme Mg²⁺ intervient avantageusement en continu dans tout le tractus gastro-intestinal de l'estomac au gros intestin, alors que l'absorption du magnésium (toujours sous la forme Mg²⁺) se fait tout le long du tractus intestinal du duodénum au gros intestin, l'absorption étant maximale au niveau de l'iléon (i. e. la dernière partie de l'intestin grêle).

Selon un aspect particulier de l'invention, on propose une composition pour administration orale de magnésium, sous forme de comprimé, avec libération progressive, une composition qui comprend
- une matrice formant un noyau comprenant :
   (A) 90 à 110 parties en poids de magnésium, la source de magnésium étant présente sous la forme d'un hydrate de formule MgCl₂.n(H₂O) où n est un nombre réel supérieur à 0 et inférieur ou égal à 6, de préférence n est compris entre 2 et 6, et mieux de 3 à 11/2, avantageusement n est 9/2,
   (B1) 180 à 190 parties en poids d'hydroxypropylméthylcellulose,
   (B2) 19,8 à 22,2 parties en poids de béhénate de glycéryle,
   (C1) 10 à 12 parties en poids de lactose, et
   (C2) 10 à 12 parties en poids de silice colloïdale ; et,
- un enrobage de protection ralentissant la libération du magnésium au niveau gastrique, qui n'est pas gastrorésistant.

### Description détaillée de l'invention

Comme l'absorption du magnésium se fait tout le long du tractus intestinal, du duodénum au gros intestin, et est optimale au niveau de l'iléon (i. e. la dernière partie de l'intestin grêle), la durée du transit variant avec le type de repas, et comme dans le système de dissolution A précité à T = 2h il reste du Mg administré non encore dissous, une quantité absorbable de manière optimale du magnésium libéré (sous la forme Mg²⁺) parvient selon l'invention au niveau de l'iléon pour traverser la paroi intestinale.

On a selon l'invention une cinétique de dissolution selon laquelle la libération de Mg intervient de façon (i) relativement lente et (ii) progressive dès la phase 'gastrique'. Cette cinétique présente une dissolution particulière au niveau de l'estomac (ce qui interdit un pelliculage gastrorésistant), d'une part, et au niveau de l'intestin grêle, d'autre part.

La matrice selon l'invention et son enrobage ne comportent aucun produit prohibé par les réglementations européennes et internationales relatives aux compléments alimentaires. En particulier, ladite matrice et ledit enrobage sont dépourvus de PCV et de polyvinylpyrrolidone.

La substance B1 est l'hydroxypropylméthylcellulose (HPMC). Elle est utilisée ici suivant une qualité qui convient pour usage pharmaceutique ou alimentaire.

La substance B2 est le béhénate de glycéryle, qui est un mélange essentiellement constitué de monoglycéride et de diglycéride de l'acide béhénique (autre nomenclature: 'mono-diglycéride béhénate') et connu sous la dénomination européenne d'additif 'E471 '. La substance B2 est également utilisée ici suivant une qualité qui convient pour usage pharmaceutique ou alimentaire.

Selon l'invention, le rapport pondéral B1/B2 est compris entre 180/22,2 = 8,1/1 et 190/19.8 = 9,6/1. Avantageusement, on recommande que ledit rapport pondéral soit compris entre 8,5/1 et 9,3/1. De préférence, le rapport pondéral B1/B2 se situera entre 8,7/1 et 9,2/1, par exemple : 8,8/1 ou 9/1 ou encore 9,15/1.

Le lactose, composant C1, est avantageusement anhydre. De même la silice colloïdale, composant C2, est avantageusement anhydre. En pratique, il est plutôt préféré que dans la matrice de l'invention le rapport pondéral C1/C2 soit voisin de 1/1 et mieux égal à 1/1.

La composition comprenant le magnésium selon l'invention peut correspondre à :
(I) des comprimés à une couche (dits 'comprimés monocouches') contenant la totalité de la source de magnésium, et
(II) des comprimés à deux couches (dits 'comprimés bicouches') comprenant
   (a) une première couche (ou couche ou noyau 'interne') gastrorésistante, ou logée dans une enveloppe gastrorésistante, ladite première couche contenant 80 à 40 % du magnésium apporté par la source de magnésium, et
   (b) une seconde couche (ou couche 'externe') qui est hydrophile, qui se dissout au niveau de l'estomac et contient 20 à 60 % du magnésium apporté par la source de magnésium.

Les comprimés utilisés selon l'invention comprennent en outre un enrobage qui n'est pas gastrorésistant. Il s'agit d'un pelliculage qui intervient (i) pour protéger les composants du comprimé nu vis-à-vis de l'extérieur, notamment vis-à-vis des chocs, et surtout (ii) pour ralentir la dissolution du Mg dans la phase 'gastrique'. Ce pelliculage peut être réalisé en une seule couche, deux couches, voire même trois couches. Pour limiter les coûts de fabrication, il est possible qu'il soit monocouche. Toutefois, un enrobage à deux couches est recommandé, pour mieux contrôler la dissolution du Mg. Comme indiqué plus haut, l'enrobage de la matrice représente en général 15 à 75 parties en poids pour une source de magnésium fournissant 90 à 110 parties en poids de Mg (i. e. approximativement 1,3 à 7,5 % en poids par rapport au poids de la matrice). De préférence il représentera 15 à 70 parties en poids, et mieux 15 à 45 parties en poids, pour 90 à 110 parties en poids de Mg.

Les substances recommandées ici pour l'enrobage sont la gomme laque, et les éthers de cellulose filmogènes tels que les alkylcelluloses, à savoir plus particulièrement les mélanges de HPMC et d'hydroxypropylcellulose (HPC) commercialisés notamment sous les nomenclatures de NUTRATERIC^{®} et OPADRY^{®}. On peut également envisager un enrobage constitué d'une première couche de gomme laque et d'une couche externe faite d'un mélange d'alkylcelluloses.

En pratique, on préconise un enrobage qui est
(a) un pelliculage monocouche de gomme laque (utilisée à 50 % en poids dans de l'éthanol, le solvant étant éliminé lors du pelliculage), ou
(b) un pelliculage à deux couches, chaque couche comprenant une substance choisie parmi la gomme laque, les éthers de cellulose (notamment HPMC et HPC) et leurs mélanges.

Quand on utilise un enrobage à deux couches, la première couche (ou couche interne) représente en général 0.5 à 4 % en poids par rapport au poids de la matrice, et la seconde couche (ou couche externe) représente en général 0,5 à 3.5 % en poids par rapport au poids de ladite matrice, l'ensemble des deux dites couches représentant 1,3 à 7,5 % en poids par rapport au poids de ladite matrice.

Selon l'invention, on préconise une composition, sous la forme comprimé pelliculé, qui libère du magnésium de façon progressive et continue. Cette composition est avantageusement constituée :
- d'une matrice constituant un noyau comprenant
   (A) 90 à 110 parties en poids de magnésium, la source de magnésium étant MgCl₂.nH₂O, où n est un nombre réel supérieur à 0 et inférieur ou égal à 6, de préférence n est compris entre 2 et 6, et mieux de 3 à 11/2, avantageusement n est 9/2,
   (B1) 180 à 190 parties en poids d'hydroxypropylméthylcellulose,
   (B2) 19,8 à 22,2 parties en poids de béhénate de glycéryle,
   (C1) 10 à 12 parties en poids de lactose, et
   (C2) 10 à 12 parties en poids de silice colloïdale ; et
- d'un pelliculage de
   (D) 15 à 45 parties en poids d'une substance choisie parmi la gomme laque, les éthers de cellulose (notamment HPMC et HPC) et leurs mélanges.

On recommande de conserver la composition utilisée selon l'invention à une température inférieure à 40 °C, et de préférence à une température inférieure ou égale à 25 °C.

La composition comprenant le magnésium est utilisée selon l'invention en association avec une composition destinée à traiter le diabète de type 2 ou ses complications.

La composition destinée à traiter le diabète de type 2 ou ses complications est selon un mode particulier de l'invention une composition pour une administration orale.

Elle comprend dans un milieu pharmaceutiquement acceptable au moins un actif pharmaceutique destiné à traiter le diabète de type 2, ou ses complications.

L'actif pharmaceutique destiné à traiter le diabète de type 2 peut être notamment choisi parmi les agents stimulant la sécrétion d'insuline, les sensibilisateurs à l'insuline, les agents diminuant la glucogénèse, les inhibiteurs de la dipeptidylpeptidase 4 et les inhibiteurs d'alpha-glucosidase.

Les agents stimulant la sécrétion d'insuline peuvent être choisis notamment parmi les sulfonylurées et les « glinides ». A titre d'exemple de sulfonylurées, on peut citer en particulier la carbutamide (Glucidoral®), le glibenclamide/glyburide (Daonil®, Euglucan®), le glibomuride (Glutril®), le gliclazide (Diamicron®), le glimépiride (Amarel®), le glipizide (Glibénèse®). A titre d'exemple de « glinides », on peut citer en particulier le répaglinide (NovoNorm®).

Les agents diminuant la glucogénèse sont généralement représentés par les biguanides, et on peut citer en particulier la metformine (Glucophage®, Stagid®).

Parmi les inhibiteurs de la dipeptidylpeptidase 4, on peut citer notamment la saxagliptine, la sitaglyptine, et la vidagliptine.

Les sensibilisateurs à l'insuline sont représentés principalement par les thiazolidinediones (TZD). On peut citer en particulier la pioglitazone (Actos®) ou la rosiglitazone (Avandia®).

Parmi les inhibiteurs d'alpha-glucosidase, on peut citer en particulier l'acarbose (Glucor®) ou le miglitol (Diastabol®).

Les patients diabétiques sont par ailleurs connus pour être une population à risque concernant le développement de pathologies cardiovasculaires, en particulier l'athérosclérose. Ceci est dû en partie à une plus grande susceptibilité à des facteurs tels que l'hyperlipidémie ou l'hypercholestérolémie. Ainsi, la diminution du taux de cholestérol à lipoprotéine basse densité (LDL-cholestérol) dans le sérum est à ce titre la première approche thérapeutique. Il peut être aussi important d'identifier les patients ayant un faible taux de cholestérol à lipoprotéine haute densité (HDL-cholestérol) et/ou des taux élevés de triglycérides. Il a en particulier été montré que les lipoprotéines riches en triglycérides provenant soit du foie (VLDL) soit de l'intestin (chylomicron) présentaient un risque athérogène important.

Selon un mode particulier de l'invention, la composition destinée à traiter le diabète de type 2 ou ses complications peut comprendre un actif pharmaceutique choisi parmi les composés diminuant le taux de lipides ou de cholestérol, tels que notamment les agonistes de PPARalpha, en particulier les fibrates (par exemple : le fénofibrate, le bézafibrate, le ciprofibrate, ou le gemfibrozyl), les inhibiteurs de HmGCoA (Hydroxyméthylglutaryl Coenzyme A réductase), tels que les statines (par exemple : atorvastatin, simvastatin, ou fluvastatin), les inhibiteurs d'absorption du cholestérol (par exemple : ezetimibe, ou les phytostérols), les inhibiteurs de CETP (Cholesteryl Ester Transfer Protein), comme par exemple torcetrapib, les inhibiteurs de l'ACAT (AcylCoA-Cholestérol Acyl Transférase), les inhibiteurs de MTP (Microsomal Triglycéride Transfer Protéine), les agents séquestrants des acides biliaires (cholestyramine), etc.

Selon un autre mode particulier de l'invention, la composition destinée à traiter le diabète de type 2 ou ses complications peut comprendre un actif pharmaceutique choisi parmi les agents anti-hypertenseurs et d'hypotension, comme les inhibiteurs d'ACE (Angiotensin-Converting Enzyme) (comme par exemple : captopril, enalapril, ramipril ou quinapril), les antagonistes des récepteurs de l'angiotensine II (par exemple : le losartan, valsartan, telmisartan, eposartan, irbesartan, etc.), les beta bloquants (aténolol, metoprolol, labétalol, propranolol), les diurétiques (par exemple : furosemide, indapamide, hydrochlorthiazide, ou anti-aldosterone), les vasodilatateurs comme les agents bloquant le récepteur alpha (comme le prazosin ou l'urapidil) ou encore le minoxidil, les agents bloquant le canaux calciques (comme par exemple ; nifedipine, felodipine, amlodipine, diltizem ou verapamil), etc.

La composition destinée à traiter le diabète de type 2 ou ses complications peut comprendre au moins deux actifs pharmaceutiques destinés à traiter le diabète de type 2 ou ses complications. Ainsi, une composition destinée à traiter le diabète de type 2 ou ses complications peut comprendre, dans la même forme pharmaceutique ou sous des formes pharmaceutiques séparées, la metformine et une sulfonylurée, une biguanide ou une thiazolidinedione. On peut citer comme exemple de combinaisons d'actifs, les produits : metformine + glibenclamide/glyburide, metformine + glipizide, metformine + pioglitazone, metformine + rosiglitazone, metformine + sitagliptine, et metformine + vidagliptine. Aussi, une composition destinée à traiter le diabète de type 2 ou ses complications peut comprendre, éventuellement dans la même forme pharmaceutique ou sous plusiquers formes pharmaceutiques, au moins un agent thérapeutique pour traiter le diabète de type 2, tel que défini ci-avant, en particulier choisi parmi les agents stimulant la sécrétion d'insuline, les sensibilisateurs à l'insuline, les agents diminuant la glucogénèse, les inhibiteurs d'alpha-glucosidase, et au moins un agent anti-hypertenseur ou d'hypotension, tel que défini ci-avant, comme les antagonistes des récepteurs de l'angiotensine II.

De nombreux produits pharmaceutiques pour traiter le diabète de type 2 sont déjà sur le marché. Ils sont généralement administrés par voie orale.

La présente invention permet donc de traiter, en particulier de diminuer les effets du diabète de type 2, et/ou de traiter au moins l'une de ses complications, en particulier les maladies cardiovasculaires, l'hypertension, la rétinopathie, la néphropathie, la dépression et/ou les ulcères du pied diabétique.

Selon un mode de l'invention, lesdites compositions sont administrées de manière simultanée, séparée ou étalée dans le temps. Les compositions peuvent être donc administrées simultanément (mais séparément), ou séquentiellement.

On entend par "séquentielle", une application séparée dans le temps de la composition comprenant le magnésium et de la composition pharmaceutique comprenant au moins un actif pharmaceutique pour traiter le diabète de type 2 ou ses complications. L'utilisateur pourra donc administrer successivement la composition comprenant le magnésium, et la composition pharmaceutique comprenant au moins un actif pharmaceutique pour traiter le diabète ou ses complications, au bout de quelques secondes ou après plusieurs heures dans la même journée notamment dans un intervalle allant de 1 heure à 3 jours. Selon une alternative, on administre en premier lieu la composition comprenant l'actif pharmaceutique pour traiter le diabète de type 2 ou ses complications, et en second lieu la composition comprenant le magnésium. Selon une autre alternative, on administre en premier lieu la composition comprenant le magnésium et en second lieu la composition pharmaceutique comprenant au moins un actif pharmaceutique pour traiter le diabète de type 2 ou ses complications.

La composition pharmaceutique comprenant au moins un actif pharmaceutique pour traiter le diabète de type 2 ou ses complications sera administrée, de préférence par voie orale, selon le traitement prescrit, et en particulier ce traitement dépendra de l'actif ou des actifs administrés et du patient.

La composition comprenant le magnésium et décrite ci-dessus comprend généralement entre 50 mg et 100 mg de magnésium. De préférence, cette composition est administrée de sorte que 100 à 500mg, de préférence 100 à 200 mg, de magnésium par jour soit administré au sujet. Avantageusement, un à dix comprimés sont administrés par jour, par exemple le matin et/ou le soir, de préférence le matin ou le matin et le soir. Selon un mode de réalisation, il peut être administré un ou deux comprimés, voire jusqu'à 4 ou 5 comprimés, une à deux fois par jour. Selon un autre mode particulier de réalisation, lorsque le comprimé comprend 50 mg de magnésium, de deux à quatre comprimés peuvent être administrés par jour, avantageusement le matin et/ou le soir, de préférence de deux à quatre comprimés uniquement le matin ou alternativement un ou deux le matin et un ou deux le soir. Selon un autre mode particulier de réalisation, lorsque le comprimé comprend 100 mg de magnésium, un à deux comprimés peuvent être administrés par jour, avantageusement le matin et/ou le soir.

Dans un mode de réalisation, le terme " traitement " ou " traiter " désigne une amélioration ou la prophylaxie du diabète de type 2 ou en particulier l'une de ses complications. Dans un autre mode de réalisation, " traitement " ou " traiter " désigne une amélioration, la prophylaxie, ou l'inversion d'au moins un paramètre physique mesurable associé à la maladie ou au trouble étant traité, qui n'est pas nécessairement discernable chez ou par le sujet traité. Dans un autre mode de réalisation, " traitement " ou " traiter " désigne l'inhibition ou le ralentissement de la progression du diabète de type 2 ou l'une de ses complications. Dans un autre mode de réalisation, "traitement" ou "traiter" désigne le retard de l'apparition d'au moins une des complications du diabète de type 2, en particulier les maladies cardiovasculaires (comme les troubles du rythme cardiaque, l'artérite, l'athérosclérose), l'hypertension, la rétinopathie, la néphropathie, la dépression ou l'ulcère du pied diabétique. En particulier, lorsqu'il s'agit de l'ulcère du pied diabétique, la présente invention peut être adaptée à la prévention, au retard de l'apparition, ou à la diminution du risque de développer un ulcère au pied diabétique.

Dans un mode de réalisation, les compositions sont administrées en tant que mesure curative. Dans le présent contexte, "curatif" désigne une réduction des effets du diabète de type 2 et/ou de diminuer le développement ou l'aggravation de l'une des complications du diabète de type 2, en particulier les maladies cardiovasculaires (comme les troubles du rythme cardiaque, l'artérite, l'athérosclérose), l'hypertension, la rétinopathie, la néphropathie, la dépression et les ulcères du pied, et plus spécifiquement les maladies cardiovasculaires (comme les troubles du rythme cardiaque, l'artérite, l'athérosclérose), l'hypertension, la rétinopathie, la néphropathie, la dépression.

La présente invention a également pour objet une méthode de traitement du diabète de type 2 ou l'une de ses complications, dans lequel on administre à un sujet atteint du diabète de type 2 et éventuellement de l'une de ses complications, une composition comprenant du magnésium telle que définie ci-dessus en association avec une composition pharmaceutique comprenant au moins un actif pharmaceutique pour traiter le diabète de type 2 ou ses complications, lesdites compositions pouvant être administrées simultanément (mais séparément), ou séquentiellement.

Au sens de la présente invention, par « patient » ou « sujet », on entend tout mammifère, et plus particulièrement les êtres humains, hommes ou femmes.

D'autres avantages et caractéristiques de l'invention seront mieux compris à la lecture des exemples suivants. Bien entendu ces exemples n'ont pas un caractère limitatif, ils sont donnés seulement à titre d'illustration.

Les essais relatifs à détermination de la cinétique de dissolution du Mg ont été réalisés à 40°C *in vitro* au moyen du système A précité : milieu HCl 0,1N de T = 0 à T = 2h, puis milieu tampon à pH 6,8 de T = 2h à T = 8h.

### Exemple 1

On a préparé des comprimés (dosés à 100 mg en magnésium) ayant la formulation suivante (le rapport pondéral B1/B2 étant de 9,15/1), Qté/cp représentant la quantité (exprimée en mg) de chaque constituant du comprimé.

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage :* | |
|---|---|
| Gomme laque | 39,6 |
| *Total* : | 989,60 |

Le profil de dissolution de ces comprimés est tel que décrit dans la présente invention.

### Exemple 2

On a préparé des comprimés (dosés à 100 mg en magnésium) ayant la formulation suivante (le rapport pondéral B1/B2 étant de 9,35/1).

| Constituants | Qté/cp (mg) |
|---|---|
| *Noyau :* | |
| MgCl₂.9/2H₂O | 725,0 |
| HPMC | 185,2 |
| Mono-diglycéride béhénate | 19,8 |
| Lactose anhydre | 11,0 |
| Silice colloïdale anhydre | 11,0 |

| *Pelliculage :* | |
|---|---|
| Gomme laque | 39,42 |
| Bleu patenté | 0,03 |
| *Total* : | 992,45 |

Le profil de dissolution de ces comprimés est tel que décrit dans la présente invention.

### Exemple 3

On a préparé des comprimés (dosés à 100 mg en magnésium) ayant la formulation suivante (le rapport pondéral B1/B2 étant de 9,25/1).

| Constituants | Qté/cp (mg) |
|---|---|
| *Noyau :* | |
| MgCl₂.9/2H₂O | 725,0 |
| HPMC | 185,0 |
| Mono-diglycéride béhénate | 20,0 |
| Lactose anhydre | 11,0 |
| Silice colloïdale anhydre | 11,0 |

| *Pelliculage* : | |
|---|---|
| Gomme laque | 39,6 |
| *Total* : | 991,60 |

Le profil de dissolution de ces comprimés est tel que décrit dans la présente invention.

### Exemple 4

On a préparé des comprimés (dosés à 100 mg en magnésium) ayant la formulation suivante, selon les modalités précitées (le rapport pondéral B1/B2 étant de 9,15/1).

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage 1* : | |
|---|---|
| Gomme laque | 24,17 |

| *Pelliculage 2* : | |
|---|---|
| mélange HPMC/HPC 1/3 p/p | 17,514 |
| Bleu patenté | 0,016 |
| *Total* : | 991,70 |

Le profil de dissolution de ces comprimés est tel que décrit dans la présente invention.

### Exemple 5

On a préparé des comprimés (dosés à 100 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage* : | |
|---|---|
| *1^{ère} couche* : Gomme laque | 19,8 |
| *2^{ème} couche (externe)* : Mélange HPMC/HPC 1/4 p/p | 19,8 |
| *Total* : | 989,60 |

Le profil de dissolution de ces comprimés est tel que décrit dans la présente invention.

### Exemple 6

On prépare selon les modalités de l'exemple 5, des comprimés renfermant 50 mg de magnésium et ayant pour chaque constituant une quantité qui est la moitié de celle du constituant homologue dudit Ex 5.

### Exemple 7

On a préparé des comprimés (dosés à 100 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 725,0 |
| HPMC | 183,0 |
| Mono-diglycéride béhénate | 20,0 |
| Lactose anhydre | 11,0 |
| Silice colloïdale anhydre | 11,0 |

| *Pelliculage* : | |
|---|---|
| *1^{ère} couche (interne)* : HPMC/HPC 1/3 p/p | 19,8 |
| *2^{ème} couche (externe)* : Mélange HPMC/HPC 1/4 p/p | 19,8 |
| *Total* : | 989,60 |

Le profil de dissolution de ces comprimés est tel que décrit dans la présente invention.

### Exemple 8

On prépare selon les modalités de l'exemple 7, des comprimés renfermant 50 mg de magnésium et ayant pour chaque constituant une quantité qui est la moitié de celle du constituant homologue dudit Ex 7.

### Exemple 9

On a préparé des comprimés (dosés à 100 mg en magnésium) ayant la formulation suivante (le rapport pondéral B1/B2 étant de 8,8/1).

| Constituants | Qté/cp (mg) |
|---|---|
| *Noyau* : | |
| MgCl₂.9/2H₂O | 725,0 |
| HPMC | 190,0 |
| Mono-diglycéride béhénate | 21,5 |
| Lactose anhydre | 10,0 |
| Silice colloïdale anhydre | 10,0 |
| Chlorhydrate de pyridoxine | 6,0 |

| *Pelliculage* : | |
|---|---|
| Gomme laque | 40,0 |
| *Total* : | 1003,0 |

### Exemple 10

On a préparé des comprimés (dosés à 50 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage 1* : | |
|---|---|
| Gomme laque (OPAGLOS^{®} NA715G, produit commercialisé par la Sté dite COLORCON) | 1,3 à 2,2 % * |

| *Pelliculage 2* : | |
|---|---|
| mélange HPMC/HPC 1/3 p/p (OPADRY^{®} VMS, produit commercialisé par la Sté dite COLORCON) | 1,1 à 1,6 % * |
| Jaune 20A38069 | 0,008 |

| | |
|---|---|
| Note (*) : pourcentage en poids par rapport au poids du comprimé nu. | |

### Exemple 11

On a préparé des comprimés (dosés à 50 mg en magnésium) ayant la formulation suivante précitée (le rapport pondéral B1/B2 étant de 9,15/1).

| Constituants | Qté/cp (mg) |
|---|---|
| *Noyau* : | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage* : | |
|---|---|
| Gomme laque (OPAGLOS^{®} NA715G, produit commercialisé par la Sté dite COLORCON) | 1,7 % * |

| | |
|---|---|
| Note (*) : pourcentage en poids par rapport au poids du comprimé nu. | |

Le profil de dissolution de ces comprimés est tel que décrit dans la présente invention.

### Exemple 12

On a préparé des comprimés (dosés à 50 mg en magnésium) ayant la formulation suivante (le rapport pondéral B1/B2 étant de 9,15/1).

| Constituants | Qté/cp (mg) |
|---|---|
| *Noyau* : | |
| MgCl₂.9/2H₂O | 362,50 |
| HPMC | 91,50 |
| Mono-diglycéride béhénate | 10,00 |
| Lactose anhydre | 5,50 |
| Silice colloïdale anhydre | 5,50 |

| *Pelliculage 1* : | |
|---|---|
| Gomme laque (OPAGLOS^{®} NA715G, produit commercialisé par la Sté dite COLORCON) | 1,7 * |

| *Pelliculage 2* : | |
|---|---|
| mélange HPMC/HPC 1/3 p/p (OPADRY^{®} VMS, | |
| produit commercialisé par la Sté dite COLORCON) | 0,5 % * |

| | |
|---|---|
| Note (*) : pourcentage en poids par rapport au poids du comprimé nu. | |

### Exemple 13

Les compositions pharmaceutiques à administration orale comprenant au moins un actif pharmaceutique pour traiter le diabète de type 2 sont par exemple choisis parmi celles citées précédemment et vendues notamment sous les marques commerciales : Glucophage ®, Metformin Biogaran®, Stagid ®, Galvus ® (DCI actif : Vidagliptine), Glucidoral ® (DCI actif : Carbutamide), Daonil (DCI actif : Glibenclamide), Amarel® (DCI actif : Glimepiride), Diamicron ® (DCI actif : Glicazide), NovoNorm® (DCI actif : répaglinide), Actos® (DCI actif : pioglitazone), Avandia® (DCI actif : rosiglitazone), Glucor® (DCI actif : acarbose), ou Diastabol® (DCI actif: miglitol), ou encore les associations d'actifs, comme par exemple Glucovance ® (DCI actifs : metformine + glibenclamide), Competact ® (DCI actifs : metformine + pioglitazone), Janumet ® (DCI actifs : metformine + sitagliptine), Eucreas ® (DCI actifs : metformine + vidagliptine).

Ces produits peuvent donc selon l'invention être administrés selon la prescription du médecin en association avec les comprimés décrits dans l'un des exemples 1-12. De préférence, ces produits contenant le magnésium sont administrés de sorte que 100 à 500 mg de magnésium par jour au sujet. Avantageusement un à dix comprimés sont administrés par jour, par exemple le matin et/ou le soir, de préférence le matin ou le matin et le soir. Selon un mode de réalisation, il peut être administré un ou deux comprimés, voire jusqu'à 4 ou 5 comprimés, une à deux fois par jour. Selon un autre mode particulier de réalisation, lorsque le comprimé comprend 50 mg de magnésium, de deux à quatre comprimés peuvent être administrés par jour, avantageusement le matin et/ou le soir, de préférence de deux à quatre comprimés uniquement le matin ou alternativement un ou deux le matin et un ou deux le soir. Selon un autre mode particulier de réalisation, lorsque le comprimé comprend 100 mg de magnésium, un à deux comprimés peuvent être administrés par jour, avantageusement le matin et/ou le soir.

## Revendications

1. Composition comprenant du magnésium à faible dose et à libération prolongée, pour une utilisation pour le traitement du diabète de type 2 ou l'une de ses complications, en association avec une composition pharmaceutique comprenant au moins un actif destiné au traitement du diabète de type 2, ladite composition comprenant du magnésium est une composition pour une administration orale, sous forme de comprimé, comprenant une matrice de magnésium avec libération progressive qui forme un noyau constitué d'un agent de retard hydrophile (B1), d'un agent de retard hydrophobe (B2), d'une charge inerte (C1) intervenant en tant que diluant et d'une charge inerte (C2) intervenant en tant que moyen lubrifiant, ladite matrice étant **caractérisée en ce qu'**elle comprend :
(A) 90 à 110 parties en poids de magnésium, la source de magnésium étant présente sous la forme choisie parmi MgO, MgCl₂ et les hydrates de formule MgCl₂.n(H₂O) où n est un nombre réel entier ou fractionnaire supérieur à 0 et inférieur ou égal à 6,
(B1) 180 à 190 parties en poids d'hydroxypropylméthylcellulose,
(B2) 19,8 à 22,2 parties en poids de béhénate de glycéryle,
(C1) 10 à 12 parties en poids de lactose, et
(C2) 10 à 12 parties en poids de silice colloïdale ;
ladite composition comprend également un enrobage, qui n'est pas un enrobage gastrorésistant, mais un enrobage de protection ralentissant la dissolution du Mg au niveau gastrique

2. Composition pour une utilisation selon la revendication 1, **caractérisée en ce que** ledit enrobage est un pelliculage à une ou plusieurs couches.

3. Composition pour une utilisation selon la revendication 2, **caractérisée en ce que** ledit enrobage représente 1,3 à 7,5 % en poids par rapport au poids de la matrice

4. Composition pour une utilisation selon la revendication 3, **caractérisée en ce que** ledit enrobage est
(a) un pelliculage monocouche de gomme laque, ou
(b) un pelliculage à deux couches, chaque couche comprenant une substance choisie parmi la gomme laque, les éthers de cellulose (notamment HPMC et HPC) et leurs mélanges.

5. Composition pour une utilisation selon l'une des revendications précédentes, ladite composition comprenant le magnésium correspond à :
(1) des comprimés à une couche (dits 'comprimés monocouches') contenant la totalité de la source de magnésium, et
(II) des comprimés à deux couches (dits 'comprimés bicouches') comprenant
(a) une première couche (ou couche ou noyau 'interne') gastrorésistante, ou logée dans une enveloppe gastrorésistante, ladite première couche contenant 80 à 40 % du magnésium apporté par la source de magnésium, et
(b) une seconde couche (ou couche 'externe') qui est hydrophile, qui se dissout au niveau de l'estomac et contient 20 à 60 % du magnésium apporté par la source de magnésium.

6. Composition pour une utilisation selon l'une des revendications précédentes, ladite composition étant constituée de :
• d'une matrice constituant un noyau comprenant
(A) 90 à 110 parties en poids de magnésium, la source de magnésium étant MgCl₂.nH₂O, où n est un nombre réel supérieur à 0 et inférieur ou égal à 6, de préférence n est compris entre 2 et 6, et mieux de 3 à 11/2, avantageusement n est 9/2,
(B1) 180 à 190 parties en poids d'hydroxypropylméthylcellulose,
(B2) 19,8 à 22,2 parties en poids de béhénate de glycéryle,
(C1) 10 à 12 parties en poids de lactose, et
(C2) 10 à 12 parties en poids de silice colloïdale ; et
• d'un pelliculage de
(D) 15 à 45 parties en poids d'une substance choisie parmi la gomme laque, les éthers de cellulose (notamment HPMC et HPC) et leurs mélanges.

7. Composition pour une utilisation selon l'une des revendications précédentes, lesdites compositions étant administrées de manière simultanée, séparée ou étalée dans le temps.

8. Composition pour une utilisation selon l'une des revendications précédentes, la composition destinée à traiter le diabète de type 2 ou ses complications comprenant dans un milieu pharmaceutiquement acceptable au moins un actif pharmaceutique destiné à traiter le diabète de type 2, ledit actif étant choisi parmi les agents stimulant la sécrétion d'insuline, les sensibilisateurs à l'insuline, les agents diminuant la glucogénèse, les inhibiteurs de la dipeptidylpeptidase 4 et les inhibiteurs d'alpha-glucosidase.

9. Composition pour une utilisation selon l'une des revendications précédentes, la composition destinée à traiter le diabète de type 2 ou ses complications comprenant au moins deux actifs pharmaceutiques destinés à traiter le diabète de type 2 ou ses complications.

10. Composition pour une utilisation selon l'une des revendications précédentes, en vue d'une diminution des effets du diabète de type 2, et/ou d'un traitement d'au moins l'une de ses complications, en particulier les maladies cardiovasculaires, l'hypertension, la rétinopathie, la néphropathie, la dépression et/ou les ulcères du pied diabétique.

11. Composition pour une utilisation selon l'une des revendications précédentes, en vue de retarder l'apparition d'au moins une des complications du diabète de type 2, en particulier les maladies cardiovasculaires (comme les troubles du rythme cardiaque, l'artérite, l'athérosclérose), l'hypertension, la rétinopathie, la néphropathie, l'ulcère du pied diabétique ou la dépression.

12. Composition pour une utilisation selon l'une des revendications précédentes, en vue de diminuer le développement ou l'aggravation de l'une des complications du diabète de type 2, en particulier les maladies cardiovasculaires (comme les troubles du rythme cardiaque, l'artérite, l'athérosclérose), l'hypertension, la rétinopathie, la néphropathie, la dépression et les ulcères du pied, et plus spécifiquement les maladies cardiovasculaires (comme les troubles du rythme cardiaque, l'artérite, l'athérosclérose), l'hypertension, la rétinopathie, la néphropathie, la dépression.

13. Composition pour une utilisation selon l'une des revendications précédentes, la composition comprenant le magnésium comprend de 50 mg à 100 mg de magnésium par comprimé.

14. Composition pour une utilisation selon l'une des revendications précédentes, la composition comprenant le magnésium étant administrée de sorte que 100 à 500 mg, de préférence 100 à 200 mg, de magnésium par jour soit administré au sujet.

15. Composition pour une utilisation selon la revendication précédente, pour une administration d'un à dix comprimés par jour, en particulier le matin et/ou le soir, de préférence le matin ou le matin et le soir.
